# EUROPEAN PATENT APPLICATION

(11) **EP 4 257 698 A1**
(43) Date of publication of application: **11.10.2023**
(21) Application number: 22831777.2
(22) Date of filing: 21.06.2022
(51) Int. Cl.: C12P 19/30, C12P 19/32, C12R 1/84, C12R 1/865

(54) **ADENOSINE-INVOLVED FULLY ENZYMATIC SYNTHESIS METHOD FOR NMN**

(30) Priority: 29.06.2021 CN 202110728796
(71) Applicant: RUBYBERRIES BIOTECHNOLOGIES CO., LTD, Zhongshan, Guangdong 528400 (CN)
(72) Inventor: JIN, Caike, Zhongshan, Guangdong 528400 (CN); ZHAO, Yuan, Zhongshan, Guangdong 528400 (CN)
(74) Representative: Tahtadjiev, Konstantin
(86) International application number: PCT/CN2022/100139
(87) International publication number: WO 2023/273959

(57) **Abstract**

An adenosine-involved fully enzymatic synthesis method for NMN includes the steps under the same reaction system: (A) reacting, under the catalytic action of a yeast cell, adenosine, phosphate and sugar that is metabolizable by a yeast cell, so as to generate ATP; and (B) generating NMN by an enzymatic reaction including the step of under the action of NAMPT, reacting nicotinamide, PRPP and ATP to generate NMN, ADP and phosphate. In this way, a series of reactions such as the generation (regeneration) of ATP, the synthesis of NMN and the utilization of ATP are performed in one reaction system in a unified manner, and thus efficient synthesis of NMN can be implemented.

## Description

### BACKGROUND OF THE PRESENT INVENTION

### FIELD OF INVENTION

The present invention relates to the technical field of the synthesis of beta-nicotinamide mononucleotide (NMN), and more particularly to an adenosine-involved fully enzymatic synthesis method for NMN.

### DESCRIPTION OF RELATED ARTS

β-nicotinamide mononucleotide (NMN) is the direct precursor for a human body to synthesize nicotinamide adenine dinucleotide (NAD). Supplementing NMN is the most effective way to increase the NAD level in the human body, it is beneficial for the metabolism of the human body and has broad and far-reaching health implications. Since the NAD levels in the elderly people are relatively low and sufficient NAD cannot be obtained from food, NMN is expected to become a dietary supplement for large-scale applications.

Currently, the conventional synthesis techniques of NMN include four methods: fermentation method, chemical synthesis method, semi-synthesis method and fully enzymatic method.Among them, the fermentation method needs to construct and produce NMN microbial strains, and in the process of mass culture and reproduction of the microorganisms, NMN is synthesized by bacterial cells. Because the basal activity of the key enzyme (NAMPT, nicotinamide phosphoribosyltransferase) that catalyzes the synthesis of NMN in various species, including low-level unicellular organisms, is generally very low, it is extremely difficult to construct a bacterial strain that highly expresses NMN. And because of the synthetic route is long and involves a multi-enzyme system and a natural decomposing enzyme system, so it is very difficult to produce NMN by efficient large-scale fermentation, and the process cost is high, and the product has no market competitiveness. The chemical synthesis method employs basic raw materials such as nicotinamide (or nicotinic acid), tetraacetyl ribose, and triphenoxyphos to first synthesize nicotinamide ribose (NR) by chemical methods, and then further phosphorylate NR to obtain NMN. The main problem of this method is that the chemical phosphorylation step of the second step involves inflammable, explosive and highly toxic substances, so that large-scale industrialization faces serious environmental protection and safety supervision problems, and there are also chemical enantiomer impurities, toxic residues of raw materials and solvents etc. The long-term safety concerns of human body application of its products are problems that are difficult to eliminate for consumers. The semi-synthetic method is to phosphorylate NR by enzymatic method on the basis of chemical synthesis of NR to obtain NMN. This method has the advantages and disadvantages of both chemical and enzymatic methods. The main problem is the residual risk of solvents and toxic components in the conventional chemical method, and the enzymatic phosphorylation step also requires expensive adenosine triphosphate (ATP), which is expensive. The fully enzymatic method uses nicotinamide, ribose and ATP as the basic raw materials, and uses a series of enzymes to catalyze the formation of NMN. The advantages of this method are environmental protection and safety, but the difficulty is that it involves the expression, purification and immobilization of various enzymes, and the cost of enzymes is high, another big problem with the fully enzyme method is that the amount of ATP is too large, which leads to the high cost of this method can becomes the main factor that hinder the fully enzymatic method to be promoted and used.

Among the four conventional synthetic methods of NMN, the fully enzymatic method imitates the natural synthesis method in the human body, and its advantages in terms of safety and environmental protection are obvious. If the manufacturing cost is appropriately reduced, the fully enzymatic method should be the most competitive in the market method.The method uses nicotinamide, ribose, ATP, etc. as the main raw materials to produce NMN through a three-step enzymatic reaction: ① Ribokinase (RK) catalyzes the conversion of ribose into 5-phosphate ribose, ② 5-phosphate ribose is catalyzed by phosphoribosyl pyrophosphotransferase (RPPK), so as to be converted into 5-phosphoribose 1-pyrophosphate (PRPP), and ③PRPP combines with nicotinamide (NAM) to generate NMN under the action of nicotinamide phosphoribosyltransferase (NAMPT). The three-step reactions of the fully enzymatic method all need to consume ATP. ATP is used as a substrate to provide phosphate groups in the first two steps of the reaction, and the products are ADP and AMP respectively; in the third step, ATP is hydrolyzed to provide energy, and the product is ADP and phosphate. The price of ATP is expensive, in order to reduce the consumption of ATP, the enzymatic reaction of coupling ATP reuse in production is needed: AMP→ADP→ATP, these two step reactions need two specific enzyme catalysis, as well as need polyphosphate (pyroxine phosphate or tripolyphosphate or hexametaphosphate, etc.) to provide phosphate groups for the substrate, and the resulting product also accumulates phosphate in addition to ATP. The accumulation of a large amount of phosphate will affect the subsequent reaction, so the process is equipped with a step for removing the phosphate, which also adversely affects the recovery rate of ATP.

### SUMMARY OF THE PRESENT INVENTION

The invention is advantageous in that it provides provide an adenosine-involved fully enzymatic synthesis method for NMN, wherein the adenosine-involved fully enzymatic synthesis method for NMN maintains the advantage of conventional fully enzymatic method and at the same time, it can simplify the purification process of NMN products and reduce the cost of raw materials, so that it has a relatively low production cost.

Another advantage of the present invention is to provide an adenosine-involved fully enzymatic synthesis method for NMN, wherein compared with the conventional fully enzymatic method, the adenosine-involved fully enzymatic synthesis method for NMN of the present invention adopts cheap adenosine instead of ATP, and the way of introducing yeast cells to convert adenosine into ATP according to energy metabolism in the reaction can be combined with the conventional fully enzymatic method to realize the reuse of ATP without the recovery process of ATP, and the phosphoric salt formed by the conventional fully enzymatic method is used as a reactant, so as to eliminate the phosphate removal process, thus simplifying the purification process of NMN products based on the participation of the adenosine.

Another advantage of the present invention is to provide an adenosine-involved fully enzymatic synthesis method for NMN, wherein compared with the conventional fully enzymatic method, the adenosine-involved fully enzymatic synthesis method for NMN of the present invention adopts cheap adenosine instead of ATP, and yeast cells are introduced in the reaction to convert adenosine into ATP according to energy metabolism, so that it can combine the conventional enzymatic method to realize the reuse of ATP and reduce the consumption of ATP raw materials, and because the price of the adenosine is much lower than that of ATP, the raw material cost of the corresponding NMN product is significantly reduced, so that it has a relatively low production cost.

Another advantage of the present invention is to provide an adenosine-involved fully enzymatic synthesis method for NMN, wherein compared with the conventional fully enzymatic method, the adenosine-involved fully enzymatic synthesis method for NMN of the present invention adopts cheap adenosine instead of ATP, and yeast cells are introduced in the reaction to convert adenosine into ATP according to energy metabolism, so that it can combine the conventional enzymatic method to realize the reuse of ATP and the phosphate formed by the conventional conventional fully enzymatic method as a reactant, that is, after separating and purifying NMN, the remaining reactants and resultants can be reused to reduce emissions, and the production of corresponding NMN products is environmentally friendly and has low environmental costs.

Another advantage of the present invention is to provide an adenosine-involved fully enzymatic synthesis method for NMN, wherein the adenosine-involved fully enzymatic synthesis method for NMN of the present invention adopts nicotinamide, ribose, adenosine, phosphate and carbohydrate that can be metabolized by yeast cells are used as raw materials, and RK, RPPK, NAMPT and yeast cells are used as catalysts to unify the generation of ATP, the synthesis of NMN and the utilization of ATP in one reaction system to complete the efficient synthesis of NMN. While improving the synthesis efficiency of the product, various reactants can be basically consumed to reduce emissions. Therefore, compared with the conventional fully enzymatic method, it is simple and easy to implement, and the cost is low.

According to an aspect of the present invention, the present invention provides an adenosine-involved fully enzymatic synthesis method for NMN which comprises the following steps in a same reaction system:
(A) generating ATP by the reaction of adenosine, phosphate and carbohydrate which is capable of being metabolized by yeast cells under the catalysis of the yeast cells; and
(B) producing NMN by enzymatic reaction, comprising the step of reacting nicotinamide, PRPP and ATP to produce NMN, ADP and phosphate under the action of NAMPT.

In one embodiment, the step (B) further comprises a step of reacting 5-ribose-phosphate and ATP to produce PRPP and AMP under the catalysis of RPPK.

In one embodiment, the adenosine-involved fully enzymatic synthesis method for NMN further comprises a step of converting AMP and phosphate into ADP under the action of the yeast cells.

In one embodiment, the step (B) further comprises a step of reacting ribose and ATP to produce 5-phosphate ribose and ADP under the catalysis of RK.

In one embodiment, in the reaction system of the adenosine-involved fully enzymatic synthesis method for NMN, RK, RPPK and NAMPT exist in at least one original form of liquid enzyme form and immobilized enzyme form.

In one embodiment, in the reaction system of the adenosine-involved fully enzymatic synthesis method for NMN, the molar ratio of adenosine to ribose ranges from 0.01 to 1.

In one embodiment, in the reaction system of the adenosine-involved fully enzymatic synthesis method for NMN, the molar ratio of ribose to phosphate ranges from 1 to 20.

In one embodiment, the adenosine-involved fully enzymatic synthesis method for NMN further comprises a step of regenerating ATP by ADP and phosphate under the action of the yeast cells.

In one embodiment, in the reaction system of the adenosine-involved fully enzymatic synthesis method for NMN, the carbohydrate metabolized by yeast cells is selected from at least one of glucose, sucrose, starch and glycerol.

In one embodiment, in the reaction system of the adenosine-involved fully enzymatic synthesis method for NMN, the yeast cells are yeast cells capable of oxidative phosphorylation metabolism.

In one embodiment, in the reaction system of the adenosine-involved fully enzymatic synthesis method for NMN, the yeast cells are selected from at least one of Pichia pastoris and Saccharomyces cerevisiae.

In one embodiment, metal ion is further added to the reaction system of the adenosine-involved fully enzymatic synthesis method for NMN.

In one embodiment, in the reaction system of the adenosine-involved fully enzymatic synthesis method for NMN, the added metal ion is at least one selected from magnesium ion and manganese ion.

In one embodiment, in the reaction system of the adenosine-involved fully enzymatic synthesis method for NMN, the molar ratio of adenosine to nicotinamide ranges from 0.01 to 1.

In one embodiment, in the reaction system of the adenosine-involved fully enzymatic synthesis method for NMN, the molar ratio of nicotinamide to phosphate ranges from 1 to 20.

In one embodiment, in the reaction system of the adenosine-involved fully enzymatic synthesis method for NMN, the yeast cells are wet yeasts once being stored cryogenically.

In one embodiment, at least one organic reagent of toluene and n-butanol is further added to the reaction system of the adenosine-involved fully enzymatic synthesis method for NMN.

In one embodiment, wherein the step (A) is initiated before the step (B) to provide ATP for the reaction of the step (B), so as to form a state in which the step (A) and the step (B) are in the same reaction system to be beneficial to promote each other.

### DETAILED DESCRIPTION OF THE PREFERRED EMBODIMENT

The following description is disclosed to enable any person skilled in the art to make and use the present invention. Preferred embodiments are provided in the following description only as examples and modifications will be apparent to those skilled in the art. The general principles defined in the following description would be applied to other embodiments, alternatives, modifications, equivalents, and applications without departing from the spirit and scope of the present invention.

Those skilled in the art should understand that, in the disclosure of the present invention, terminologies of "longitudinal," "lateral," "upper," "front," "back," "left," "right," "perpendicular," "horizontal," "top," "bottom," "inner," "outer," and etc. that indicate relations of directions or positions are based on the relations of directions or positions shown in the appended drawings, which are only to facilitate descriptions of the present invention and to simplify the descriptions, rather than to indicate or imply that the referred device or element is limited to the specific direction or to be operated or configured in the specific direction. Therefore, the above-mentioned terminologies shall not be interpreted as confine to the present invention.

It is understandable that the term "a" or "an" should be understood as "at least one" or "one or more". In other words, in some embodiments, the number of an element can be one and in other embodiment the number of the element can be more than one. The term "a" or "an" is not construed as a limitation of quantity.

The present invention provides an adenosine-involved fully enzymatic synthesis method for NMN, compared with the conventional fully enzymatic method, the adenosine-involved fully enzymatic synthesis method for NMN of the present invention adopts cheap adenosine instead of ATP, and yeast cells are introduced in the reaction to convert adenosine into ATP according to energy metabolism, so that it can combine the conventional enzymatic method to realize the reuse of ATP and utilize the phosphate formed by the conventional conventional fully enzymatic method as a reactant.

More specifically, the adenosine-involved fully enzymatic synthesis method for NMN adopts nicotinamide, ribose, adenosine, phosphate, and carbohydrate that can be metabolized by yeast cells as raw materials, and uses RK, RPPK, NAMPT, and yeast cells as catalysts, the generation of ATP, the synthesis of NMN and the utilization of ATP are unified in one reaction system, and the efficient synthesis of NMN can be completed. The reaction formula is: nicotinamide + ribose + carbohydrate that can be metabolized by yeast cells + adenosine + Phosphate+O₂→NMN+ATP+CO₂+H₂O. In this reaction system, yeast cells use the dehydrogenation and oxidation of carbohydrate that can be metabolized by yeast cells to provide energy to drive the combination of phosphate and adenosine to generate adenosine monophosphate (AMP) through the metabolic process of oxidative phosphorylation, and subsequently generate ADP and ATP, then ATP activates ribose to generate PRPP, and PRPP reacts with NAM to produce NMN. The original and generated ADP, AMP, adenosine and phosphate in the reaction system can also be automatically converted into ATP by yeast cells to continue to participate in the reaction. Compared with the conventional fully enzymatic method, the formed phosphate can be used as a reactant, so that the removal process of phosphate is eliminated, and the reuse of ATP can be realized without the recovery process of ATP and the consumption of ATP raw materials is reduced, so the purification process of the NMN product is simplified based on the participation of the adenosine.

Furthermore, in one embodiment of the present invention, the adenosine-involved fully enzymatic synthesis method for NMN adopts nicotinamide, ribose, phosphate, adenosine, sucrose and magnesium ions as raw materials, and adopts RK, RPPK, NAMPT and Saccharomyces cerevisiae are used as catalysts. In aqueous solution, the initial pH is in the neutral range, and the reaction is carried out in contact with air and under stirring. Then the generation of ATP, the synthesis of NMN and the utilization of ATP are carried out in one reaction system, and various reactants can be substantially completely consumed, and the corresponding reaction system is simple and easy, with low cost, and is environmentally friendly and has low environmental cost.

In another embodiment of the present invention, nicotinamide, D-ribose and adenosine are used as substrates, and Saccharomyces cerevisiae, RK magnetic immobilized enzyme, RPPK magnetic immobilized enzyme and NAMPT magnetic immobilized enzyme are used for one-pot production of NMN. Add adenosine with a final concentration of 50mM, 330mM dipotassium hydrogen phosphate, 70mM potassium dihydrogen phosphate, 120mM sucrose, 50mM magnesium chloride, 5mM manganese chloride, and 300g Saccharomyces cerevisiae in 1L reaction system. After fully stirring and dissolving, control the reaction temperature to be 37° C, and the fermentation is carried out for one hour. Add nicotinamide with a final concentration of 100mM, 50mM D-ribose, 100g ribokinase magnetically immobilized enzyme, 100g ribose phosphate pyrophosphate kinase magnetically immobilized enzyme, and 300g nicotinamide phosphoribosyltransferase magnetically immobilized enzyme in the above yeast fermentation broth Lyase (the above three magnetically immobilized enzymes are all provided by Hong Kong Life Science and Technology Research Institute Co., Ltd.), stir at 300rpm, control the reaction temperature at 37°C, and use an automatic titrator to control the reaction pH to 6.0 with 3M sodium hydroxide. During the reaction process, the NMN concentration is detected by the high performance liquid chromatography, and the reaction is completed within four hours, and 13.77 g of NMN is obtained from the reaction, and the reaction conversion rate is 82.4%.

It is worth mentioning that, in order to reduce the amount of enzyme used and simplify the complexity of the reaction, the reaction intermediates 5-phosphate ribose and PRPP involved in the present invention can also be directly put into the reaction system as the substrate (raw material) of the reaction. If ribose 5-phosphate is used instead of ribose as the raw material, ribokinase (RK) is not needed; if PRPP is used instead of ribose as the raw material, the two enzymes RK and RPPK are not needed.

In another embodiment of the present invention, the adenosine-involved fully enzymatic synthesis method for NMN adopts 5-phosphoribosyl 1-pyrophosphate, nicotinamide and adenosine as substrates, and uses yeast and nicotinamide phosphoribosyl transfererase enzyme to carry out one-pot production of NMN. Add nicotinamide with a final concentration of 100mM, 50mM 5-phosphoribosyl 1-pyrophosphate, 50mM adenosine, 330mM dipotassium hydrogen phosphate, 70mM potassium dihydrogen phosphate, 120mM Sucrose, 50mM magnesium chloride, 5mM manganese chloride, 300g Saccharomyces cerevisiae, with 300U nicotinamide phosphoribosyl transferase liquid enzyme, after fully stirring and dissolving, control the reaction temperature to 37°C, the reaction pH to 6.0, 300rpm to stir the reaction, and the concentration of NMN is detected by the high performance liquid chromatography, and the reaction is completed within four hours, and 15.44 g of NMN is obtained from the reaction, and the reaction conversion rate is 92.4%.

In another embodiment of the present invention, the adenosine-involved fully enzymatic synthesis method for NMN adopts nicotinamide, 5-phosphate ribose and adenosine as substrates, adopts Saccharomyces cerevisiae and ribose phosphate pyrophosphate kinase, nicotinamide phosphoribosyl transferase enzyme to carry out one-pot production of NMN. Add adenosine with a final concentration of 50mM, 330mM dipotassium hydrogen phosphate, 70mM potassium dihydrogen phosphate, 120mM sucrose, 50mM magnesium chloride, 5mM manganese chloride, and 300g brewing yeast into a 1L reaction system , after fully stirring and dissolving, control the reaction temperature at 37°C and ferment for one hour. Add nicotinamide with a final concentration of 100mM, 50mM ribose-5-phosphate, 300U nicotinamide phosphoribosyltransferase liquid enzyme, 300U ribose phosphate pyrophosphokinase to the above yeast fermentation broth, stir the reaction at 300rpm, and control the reaction temperature at 37°C, use an automatic titrator to control the reaction pH to 6.0 with 3M sodium hydroxide. During the reaction process, the NMN concentration is detected by the high performance liquid chromatography, and the reaction is completed within six hours, and 14.42 g of NMN is obtained from the reaction, and the reaction conversion rate is 86.3%.

To further illustrate the present invention, the adenosine-involved fully enzymatic synthesis method for NMN of the present invention comprises the following steps in the same reaction system:
(A) generating ATP by the reaction of adenosine, phosphate and carbohydrate which is capable of being metabolized by yeast cells under the catalysis of the yeast cells; and
(B) producing NMN by enzymatic reaction, comprising the step of reacting nicotinamide, PRPP and ATP to produce NMN, ADP and phosphate under the action of NAMPT.

Furthermore, the step (B) further comprises a step of reacting 5-ribose-phosphate and ATP to produce PRPP and AMP under the catalysis of RPPK.

It is worth mentioning that the adenosine-involved fully enzymatic synthesis method for NMN further comprises a step of converting AMP and phosphate into ADP under the action of the yeast cells.

In addition, the step (B) further comprises a step of reacting ribose and ATP to produce 5-phosphate ribose and ADP under the catalysis of RK.

It is worth mentioning that in the reaction system of the adenosine-involved fully enzymatic synthesis method for NMN, RK, RPPK and NAMPT exist in at least one original form of liquid enzyme form and immobilized enzyme form.

Particularly, in the reaction system of the adenosine-involved fully enzymatic synthesis method for NMN, the molar ratio of adenosine to ribose ranges from 0.01 to 1.

Furthermore, in the reaction system of the adenosine-involved fully enzymatic synthesis method for NMN, the molar ratio of ribose to phosphate ranges from 1 to 20.

It is worth mentioning that the adenosine-involved fully enzymatic synthesis method for NMN further comprises a step of regenerating ATP by ADP and phosphate under the action of yeast cells.

It can be understood that in the reaction system of the adenosine-involved fully enzymatic synthesis method for NMN, the carbohydrate metabolized by yeast cells is selected from at least one of glucose, sucrose, starch, glycerol, and the combination thereof.

Furthermore, in the reaction system of the adenosine-involved fully enzymatic synthesis method for NMN, the yeast cells are yeast cells capable of oxidative phosphorylation metabolism, such as Pichia pastoris and Saccharomyces cerevisiae.

Alternatively, metal ion is further added to the reaction system of the adenosine-involved fully enzymatic synthesis method for NMN, and the added metal ion can be magnesium ion or manganese ion.

Preferably, in the reaction system of the adenosine-involved fully enzymatic synthesis method for NMN, the molar ratio of adenosine to nicotinamide ranges from 0.01 to 1.

Preferably, in the reaction system of the adenosine-involved fully enzymatic synthesis method for NMN, the molar ratio of nicotinamide to phosphate ranges from 1 to 20.

Preferably in the reaction system of the adenosine-involved fully enzymatic synthesis method for NMN, the yeast cells are wet yeasts once being stored cryogenically.

Preferably, at least one organic reagent of toluene and n-butanol is further added to the reaction system of the adenosine-involved fully enzymatic synthesis method for NMN.

It is worth mentioning that, in some embodiments of the present invention, the step (A) is initiated before the step (B) to provide ATP for the reaction of the step (B), so as to form a state in which the step (A) and the step (B) are in the same reaction system to be beneficial to promote each other.

One skilled in the art will understand that the embodiment of the present invention as shown in the drawings and described above is exemplary only and not intended to be limiting.

It will thus be seen that the objects of the present invention have been fully and effectively accomplished. The embodiments have been shown and described for the purposes of illustrating the functional and structural principles of the present invention and are subject to change without departure from such principles. Therefore, this invention includes all modifications encompassed within the spirit and scope of the following claims.

## Claims

1. An adenosine-involved fully enzymatic synthesis method for NMN which comprises the following steps in a same reaction system:
(A) generating ATP by the reaction of adenosine, phosphate and carbohydrate which is capable of being metabolized by yeast cells under the catalysis of the yeast cells; and
(B) producing NMN by enzymatic reaction which comprises a step of reacting nicotinamide, PRPP and ATP to produce NMN, ADP and phosphate under the action of NAMPT.

2. The adenosine-involved fully enzymatic synthesis method for NMN according to claim 1, wherein the step (B) further comprises a step of reacting 5-ribose-phosphate and ATP to produce PRPP and AMP under the catalysis of RPPK.

3. The adenosine-involved fully enzymatic synthesis method for NMN according to claim 2, wherein the adenosine-involved fully enzymatic synthesis method for NMN further comprises a step of converting AMP and phosphate into ADP under the action of the yeast cells.

4. The adenosine-involved fully enzymatic synthesis method for NMN according to claim 2, wherein the step (B) further comprises a step of reacting ribose and ATP to produce 5-phosphate ribose and ADP under the catalysis of RK.

5. The adenosine-involved fully enzymatic synthesis method for NMN according to claim 4, wherein in the reaction system of the adenosine-involved fully enzymatic synthesis method for NMN, RK, RPPK and NAMPT exist in at least one original form of liquid enzyme form and immobilized enzyme form.

6. The adenosine-involved fully enzymatic synthesis method for NMN according to claim 5, wherein in the reaction system of the adenosine-involved fully enzymatic synthesis method for NMN, the molar ratio of adenosine to ribose ranges from 0.01 to 1.

7. The adenosine-involved fully enzymatic synthesis method for NMN according to claim 6, wherein in the reaction system of the adenosine-involved fully enzymatic synthesis method for NMN, the molar ratio of ribose to phosphate ranges from 1 to 20.

8. The adenosine-involved fully enzymatic synthesis method for NMN according to one of claims 1-7, wherein the adenosine-involved fully enzymatic synthesis method for NMN further comprises a step of regenerating ATP by ADP and phosphate under the action of the yeast cells.

9. The adenosine-involved fully enzymatic synthesis method for NMN according to claim 8, wherein in the reaction system of the adenosine-involved fully enzymatic synthesis method for NMN, the carbohydrate metabolized by the yeast cells is selected from at least one of glucose, sucrose, starch and glycerol.

10. The adenosine-involved fully enzymatic synthesis method for NMN according to claim 8, wherein in the reaction system of the adenosine-involved fully enzymatic synthesis method for NMN, the yeast cells are yeast cells capable of oxidative phosphorylation metabolism.

11. The adenosine-involved fully enzymatic synthesis method for NMN according to claim 8, wherein in the reaction system of the adenosine-involved fully enzymatic synthesis method for NMN, the yeast cells are selected from at least one of Pichia pastoris and Saccharomyces cerevisiae.

12. The adenosine-involved fully enzymatic synthesis method for NMN according to claim 8, wherein metal ion is further added to the reaction system of the adenosine-involved fully enzymatic synthesis method for NMN.

13. The adenosine-involved fully enzymatic synthesis method for NMN according to claim 12, wherein in the reaction system of the adenosine-involved fully enzymatic synthesis method for NMN, the added metal ion is at least one selected from magnesium ion and manganese ion.

14. The adenosine-involved fully enzymatic synthesis method for NMN according to claim 8, wherein in the reaction system of the adenosine-involved fully enzymatic synthesis method for NMN, the molar ratio of adenosine to nicotinamide ranges from 0.01 to 1.

15. The adenosine-involved fully enzymatic synthesis method for NMN according to claim 8, wherein in the reaction system of the adenosine-involved fully enzymatic synthesis method for NMN, the molar ratio of nicotinamide to phosphate ranges from 1 to 20.

16. The adenosine-involved fully enzymatic synthesis method for NMN according to claim 8, wherein in the reaction system of the adenosine-involved fully enzymatic synthesis method for NMN, the yeast cells are wet yeasts once being stored cryogenically.

17. The adenosine-involved fully enzymatic synthesis method for NMN according to claim 8, wherein at least one organic reagent of toluene and n-butanol is further added to the reaction system of the adenosine-involved fully enzymatic synthesis method for NMN.

18. The adenosine-involved fully enzymatic synthesis method for NMN according to claim 8, wherein the step (A) is initiated before the step (B) to provide ATP for the reaction of the step (B), so as to form a state in which the step (A) and the step (B) are in the same reaction system to be beneficial to promote each other.
